# EUROPÄISCHE PATENTSCHRIFT

(11) **EP 0 627 413 B1**
(45) Veröffentlichungstag und Bekanntmachung des Hinweises auf die Patenterteilung: **24.09.1997**
(21) Anmeldenummer: 94108281.0
(22) Anmeldetag: 30.05.1994
(51) Int. Cl.: C07C 279/22

(54) **Substituierte Benzonylguanidine, Verfahren zu ihrer Herstellung, ihre Verwendung als Medikament oder Diagnostikum sowie sie enthaltendes Medikament**
Substituted benzoylguanidines, process for their preparation, their use as medicaments as well as medicament containing them
Benzoylguanidines, procédé de leur préparation, leur utilisation comme médicaments ainsi que médicaments les contenant

(30) Priorität: 04.06.1993 DE 4318658
(43) Veröffentlichungstag der Anmeldung: 07.12.1994
(73) Patentinhaber: HOECHST AKTIENGESELLSCHAFT, 65929 Frankfurt am Main (DE)
(72) Erfinder: Lang, Hans-Jochen, Dr., D-65719 Hofheim/Ts. (DE); Kleemann,Heinz-Werner, Dr., D-61350 Bad Homburg (DE); Scholz, Wolfgang, Dr., D-65760 Eschborn (DE); Albus, Udo, Dr., D-61197 Florstadt (DE)

(56) Entgegenhaltungen:
- EP-A- 0 416 499
- EP-A- 0 556 672
- EP-A- 0 556 674
- DE-A- 3 502 629

## Beschreibung

Die Erfindung betrifft Benzoylguanidine der Formel I 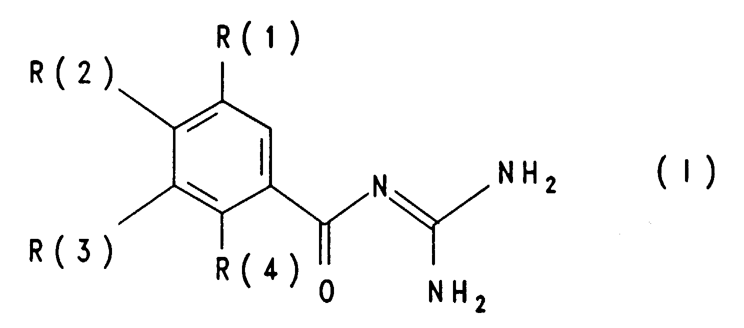 worin bedeuten:
- R(1): Wasserstoff, F, Cl, Br, I, -NO₂, -C≡N, Xₒ-(CH₂)ₚ-(CF₂)_{q}-CF₃, R(5)-SOₘ, R(6)-CO- oder R(6)R(7)N-SO₂-, mit
X gleich Sauerstoff, S, NR(14),
m gleich Null, 1 oder 2,
o gleich Null, 1,
p gleich Null, 1, 2,
q gleich Null, 1, 2, 3, 4, 5, 6,
R(5) und R(6) gleich (C₁-C₈)-Alkyl, (C₃-C₆)-Alkenyl, -CₙH₂ₙ-R(8), CF₃,
   n gleich Null, 1, 2, 3 oder 4,
   R(8) gleich (C₃-C₇)-Cycloalkyl, Phenyl, welches nicht substituiert ist oder substituiert mit 1 - 3 Substituenten aus der Gruppe
      F, Cl, CF₃, Methyl, Methoxy oder NR(9)R(10) mit R(9) und R(10) gleich H oder C₁-C₄-Alkyl,
wobei R(6) auch in der Bedeutung von H steht,
R(7) gleich H oder (C₁-C₄)-Alkyl,
wobei R(6) und R(7) gemeinsam 4 oder 5 Methylengruppen sein können, von denen eine CH₂-Gruppe durch Sauerstoff, S, NH, N-CH₃ oder N-Benzyl ersetzt sein kann,
- R(2): 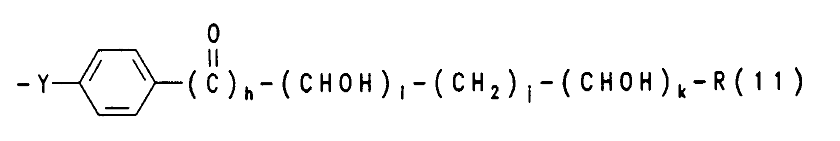 oder 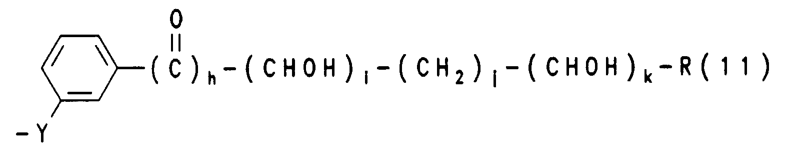 oder 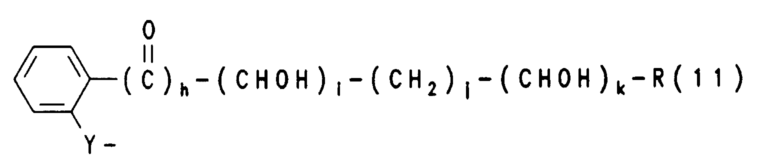 wobei Y gleich Sauerstoff, -S- oder -NR(12)- ist,
R(11), R(12) = Wasserstoff oder (C₁-C₃)-Alkyl, und
h Null oder 1, und
i, j und k unabhängig Null, 1, 2, 3 oder 4 bedeuten,
wobei jedoch nicht h, i und k gleichzeitig Null sind,
- R(3): wie R(1) definiert, oder (C₁-C₆)-Alkyl, -X-R(13) mit
X gleich Sauerstoff, S, NR(14),
R(14) gleich H, (C₁-C₃)-Alkyl,
R(13) gleich H, (C₁-C₆)-Alkyl, (C₃-C₈)-Cycloalkyl, -C_{b}H_{2b}-R(15) mit b gleich Null, 1, 2, 3 oder 4 ist und
wobei R(13) und R(14) auch gemeinsam 4 oder 5 Methylengruppen sein können und eine CH₂-Gruppe durch Sauerstoff, S, NH, N-CH₃ oder N-Benzyl ersetzt sein kann,
R(15) Phenyl, das unsubstituiert oder substituiert ist mit 1-3 Substituenten aus der Gruppe F, Cl, CF₃, Methyl, Methoxy oder NR(9)R(10) mit
   R(9) und R(10) gleich H oder (C₁-C₄)-Alkyl,
- R(4): Wasserstoff, -OR(16) oder -NR(16)R(17) mit
R(16), R(17) unabhängig Wasserstoff, (C₁-C₃)-Alkyl
sowie deren pharmazeutisch verträgliche Salze.

Bevorzugt sind Verbindungen der Formel I, in der bedeuten:
- R(1): Wasserstoff, F, Cl, -C≡N, -CF₃, R(5)-SOₘ, R(6)-CO- oder
R(6)R(7)N-SO₂-, mit
m gleich Null, 1 oder 2,
R(5) und R(6) gleich (C₁-C₈)-Alkyl, (C₃-C₄)-Alkenyl, -CₙH₂ₙ-R(8),-CF₃,
   n gleich Null oder 1,
   R(8) gleich (C₃-C₆)-Cycloalkyl, Phenyl,
      welches unsubstituiert ist oder substituiert mit 1-3 Substituenten aus der Gruppe F, Cl, CF₃, Methyl, Methoxy oder NR(9)R(10) mit R(9) und R(10) gleich H oder Methyl,
   wobei R(6) auch in der Bedeutung von H steht,
   R(7) gleich H oder Methyl.
- R(3): Wasserstoff, Methyl, Cyano, -CF₃, F, Cl
und die übrigen Reste wie oben definiert sind,
sowie deren pharmazeutisch verträgliche Salze.

Besonders bevorzugt sind Verbindungen I, in denen bedeuten:
- R(1): Wasserstoff, F, Cl, -C≡N, -CF₃, R(5)-SOₘ, R(6)-CO- oder R(6)R(7)N-SO₂-, mit
m gleich Null, 1 oder 2,
R(5) gleich Methyl oder CF₃,
R(6), R(7) unabhängig voneinander gleich H oder Methyl;
- R(2): 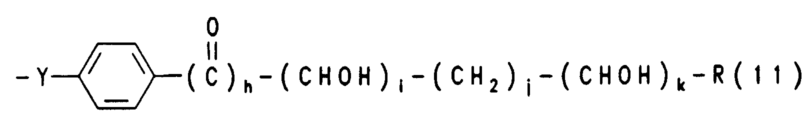 oder 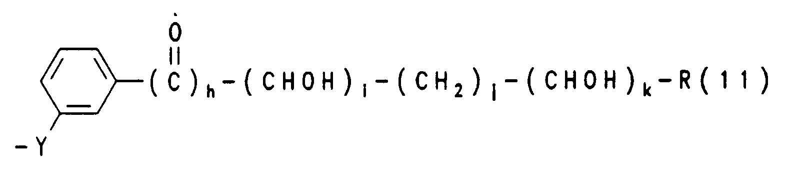 oder 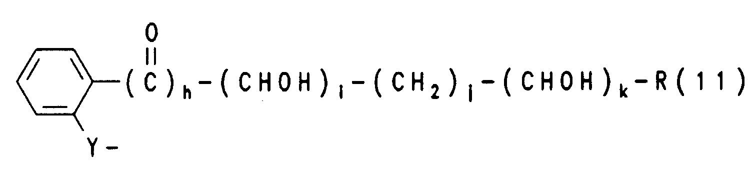
wobei Y = Sauerstoff, S oder -NR(12),
R(11), R(12) unabhängig Wasserstoff oder Methyl,
h gleich Null oder 1,
i, k unabhängig voneinander gleich Null, 1, 2 oder 3,
j gleich Null oder 1,
wobei jedoch nicht h, i und k gleichzeitig Null sind,
- R(3): Methyl, Cyano, Trifluormethyl, F, Cl oder Wasserstoff,
- R(4): Wasserstoff, OH oder NH₂
sowie deren pharmazeutisch verträgliche Salze.

Enthält einer der Substituenten R(1) bis R(4) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können sowohl geradkettig wie verzweigt vorliegen.

Die Erfindung betrifft weiterhin ein Verfahren zur Herstellung der Verbindung I, dadurch gekennzeichnet, daß man Verbindungen der Formel II 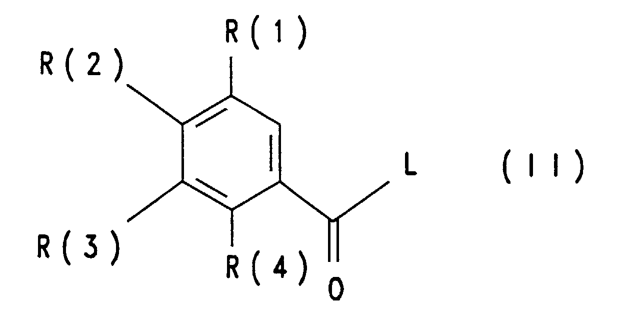 mit Guanidin umsetzt, worin R(1) bis R(4) die angegebene Bedeutung besitzen und L für eine nucleophil substituierbare Fluchtgruppe steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L=Cl), die man ihrerseits wiederum in an sich bekannter Weise aus den zugrundeliegenden Carbonsäuren (Formel II, L= OH) beispielsweise mit Thionylchlorid herstellen kann.

Neben den Carbonsäurechloriden der Formel II (L=Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L=OCH₃ durch Behandeln mit gasförmigem HCl in Methanol, die Imidazolide der Formel II durch Behandeln mit Carbonyldiimidazol [L= 1-Imidazolyl, Staab, Angew. Chem. Int. Ed. Engl. 1, 351 - 367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit

O-[(Cyano(ethoxycarbonyl)methylen)amino]-1,1,3,3-tetramethyluronium-tetraflu orborat ("TOTU")[Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = L=OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II und III verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden, indem man

### beispielsweise

4-(bzw. 5-)Halogen-3-chlor-sulfonylbenzoesäuren mit Ammoniak oder Aminen in 3-Aminosulfonyl-4-(bzw. 5-)halogen-benzoesäuren bzw. mit einem schwachen Reduktionsmittel wie Natriumbisulfit und anschließender Alkylierung in 3-Alkylsulfonyl-4-(bzw. 5-)halogen-benzoesäuren überführt und die erhaltenen Benzoesäuren nach einer der oben beschriebenen Verfahrensvarianten zu erfindungsgemäßen Verbindungen I umgesetzt werden.

Die Einführung einiger Substituenten in 4- und 5-Stellung gelingt durch literaturbekannte Methoden des Palladium-vermittelten cross-couplings von Arylhalogeniden mit beispielsweise Organostannanen, Organoboronsäuren oder Organoboranen oder Organokupfer- bzw. zink-verbindungen.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen 1 sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 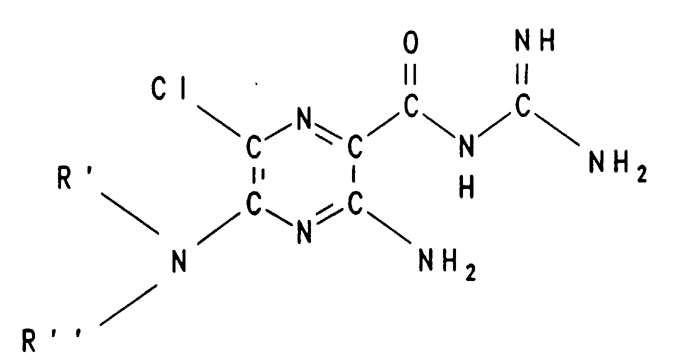 Amilorid: R',R'' = H
Dimethylamilorid: R',R'' = CH₃
Ethylisopropylamilorid: R' = C₂H₅, R'' = CH(CH₃)₂

Darüberhinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288) sind Benzoylguanidine beschrieben, die in der dem Rest R(1) entsprechenden Stellung ein Wasserstoff-Atom tragen. In der deutschen Patentanmeldung P 42 04 575.4 (HOE 92/F 034) werden 3,5-substituierte Benzoylguanidine vorgeschlagen, in welchen aber der Substituent R(2) nicht die nach der vorliegenden Erfindung beanspruchten Bedeutungen hat.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften gegen solche Arrhythmien aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den bekannten Verbindungen weisen die Verbindungen nach der Erfindung eine signifikant verbesserte Wasserlöslichkeit auf. Daher sind sie wesentlich besser für i.V.-Applikationen geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers. Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

Liste der Abkürzungen:
- MeOH: Methanol
- DMF: N,N-Dimethylformamid
- NBS: N-Bromsuccinimid
- AlBN: α,α-Azo-bis-isobutyronitril
- EI: electron impact
- DCI: Desorption-Chemical Ionisation
- RT: Raumtemperatur
- EE: Ethylacetat (EtOAc)
- DIP: Diisopropylether
- MTB: Methyltertiärbutylether
- mp: Schmelzpunkt
- HEP: n-Heptan
- DME: Dimethoxyethan
- FAB: Fast Atom Bombardment
- CH₂Cl₂: Dichlormethan
- THF: Tetrahydrofuran
- eq: Äquivalent
- ES: Elektrospray-lonisation

### Experimenteller Teil

Allgemeine Vorschrift zur Herstellung von Benzoyl-guanidinen (I)
Variante A: aus Benzoesäuren (II, L=OH)

0,01 M des Benzoesäurederivates der Formel II löst bzw. suspendiert man in 60 ml wasserfreiem THF und versetzt sodann mit 1,78 g (0,011 M) Carbonyldiimidazol. Nach dem Rühren über 2 Stunden bei RT werden 2,95 g (0,05 M) Guanidin in die Reaktionslösung eingetragen. Nach dem Rühren über Nacht destilliert man das THF unter vermindertem Druck (Rotavapor) ab, versetzt mit Wasser, stellt mit 2N HCL auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

Allgemeine Vorschrift zur Herstellung von Benzoyl-guanidinen (I)
Variante B: aus Benzoesäure-alkylestern (II, L = O-Alkyl)

5 mmol des Benzoesäure-alkylesters der Formel II sowie 25 mmol Guanidin (freie Base) werden in 15 ml Isopropanol gelöst oder in 15 ml THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotavapor) abdestilliert, in 300 ml EE aufgenommen und 3 x mit je 50 ml NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert. (Salzbildung vergleiche Variante A)

### Beispiel 1

4-(4-Acetylphenoxy)-3-methylsulfonyl-benzoylguanidin Hydrochlorid

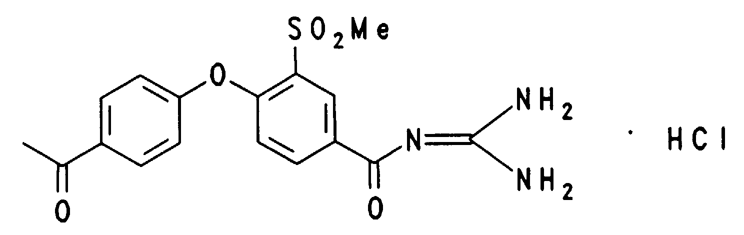

### a) 4-(4-Acetylphenoxy)-3-methylsulfonyl-benzoesäuremethylester

5 mmol 4-Chlor-3-methylsulfonyl-benzoesäuremethylester, 15 mmol K₂CO₃ sowie 5 mmol 4-Hydroxyacetophenon werden unter Argon in 10 ml DMF (wasserfrei) 2 h bei 130°C gerührt. Anschließend wird das DMF im Vakuum entfernt, der Rückstand mit 50 ml Wasser und 50 ml EE aufgenommen und 2x mit 50 ml EE extrahiert. Über Na₂SO₄ wird getrocknet und das Solvens im Vakuum entfernt. Kristallisation aus Diethylether liefert 1,2 g eines hellbraunen Feststoffs mp 135°C.
R_{f} (MTB) = 0,41 MS (DCI) : 349 (M + 1)

### b) 4-(4-Acetylphenoxy)-3-methylsulfonyl-benzoylguanidin, Hydrochlorid

1,2 mmol Ester 1a) und 6,0 mmol Guanidin werden nach der allgemeinen Vorschrift B umgesetzt. 130 g weißes Pulver mp > 270°C
R_{f} (EE/MeOH 5:1) = 0,46 MS (DCI) : 376 (M + 1)

### Beispiel 2

4-[4-(2(R,S)-Hydroxyethyl)phenoxy]-methylsulfonyl-benzoylguanidin, Hydrochlorid

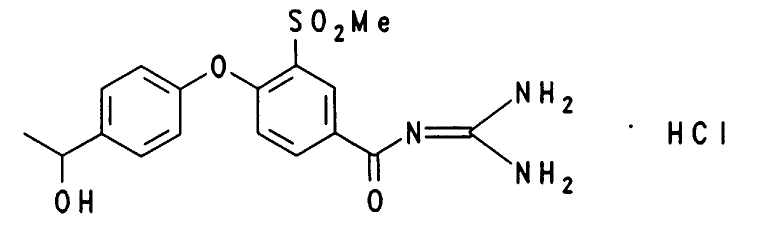

### a) 4-[4-(2(RS)-Hydroxyethyl)phenoxy-methylsulfonyl-benzoesäureethylester

2,4 mmol Keton 1a) sowie 2,4 mmol NaBH₄ werden in 10 ml Ethanol (wasserfrei) gelöst und unter Argon 20 h bei RT gerührt. Dann werden 10 ml Wasser zugegeben, und das Ethanol wird im Vakuum entfernt. Anschließend werden 10 ml gesättigte wäßrige NaCl-Lösung zugegeben, und es wird 3 x mit 20 ml EE extrahiert. Über Na₂SO₄ wird getrocknet und das Solvens im Vakuum entfernt. Man erhält 700 mg eines blaßgelben Öls.
R_{f} (MTB) = 0,41 MS (DCI) : 363 (M + 1)

### b) 4-[4-(2(R,S)-Hydroxyethyl)phenoxy]-methylsulfonyl-benzoylguanidin, Hydrochlorid

1,9 mmol Ester 2a) und 9,6 mmol Guanidin werden nach der allgemeinen Vorschrift B umgesetzt. Weißes Pulver mp > 270°C
R_{f} (EE/MeOH 10:1) = 0,21 MS (DCI) : 379 (M + 1)

### Beispiel 3

4-[4-(1(R),2(R)-Dihydroxyprop-1-yl)phenoxy]-3-methylsulfonyl-benzoylguanidin

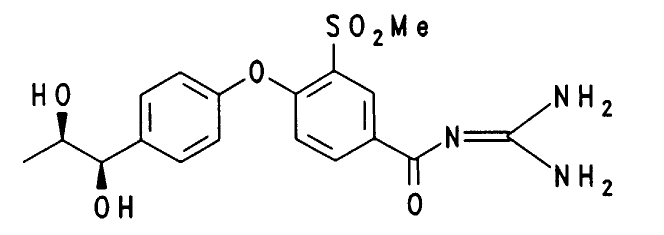

### a) 4-(4-Formylphenoxy)-3-methylsulfonyl-benzoesäuremethylester

39 mmol 4-Fluor-3-methylsulfonyl-benzoesäuremethylester, 39 mmol 4-Formylphenol sowie 78 mmol K₂CO₃ werden unter Argon in 100 ml DMF (wasserfrei) 3,5 h bei 130°C gerührt. Dann wird auf 300 ml Wasser gegossen und 3x mit je 300 ml EE extrahiert. Über Na₂SO₄ wird getrocknet und das Solvens im Vakuum entfernt. Aus MTB wird umkristallisiert, und man erhält 1,6 g farblosen Feststoff.
R_{f} (MTB) = 0,59 MS (ES) : 335 (M + 1)

### b) 4-(4-Propen-1-yl-phenoxy)-3-methylsulfonyl-benzoesäure-methylester, E/Z-Gemisch

7,2 mmol Ethyl-triphenylphosphoniumbromid werden in 50 ml THF (wasserfrei) suspendiert und bei RT mit 6,7 mmol Kalium-t-butylat versetzt. 3 h wird bei dieser Temperatur gerührt, anschließend auf 0°C gekühlt und eine Lösung von 4,8 mmol des Aldehyds 3a) in 15 ml THF zugetropft. 1 h wird bei RT gerührt, anschließend auf 50 ml gesättigte wäßrige NaCl-Lösung gegossen und 3x mit 100 ml EE extrahiert. Über Na₂SO₄ wird getrocknet und das Solvens im Vakuum entfernt. Chromatographie an Kieselgel mit DIP liefert 1,3 g farbloses Öl.
R_{f} (DIP) = 0,40 MS (ES) : 346 (M + 1)

### c) (E)-4-(4-Propen-1-yl-phenoxy)-3-methylsulfonyl-benzoesäuremethylester

3,8 mmol E/Z-Gemisch 3b) sowie 3,8 mmol Iod werden in 50 ml CH₂Cl₂ 5 Tage bei RT stehen gelassen. Man gießt auf 50 ml Na₂SO₃-Lösung und extrahiert 2x mit 50 ml CH₂Cl₂. Über Na₂SO₄ wird getrocknet und das Solvens im Vakuum entfernt. 1,3 g farbloses Öl.

### d) 4-[4-(1(R),2(R)-Dihydroxyprop-1-yl)phenoxy]-3-methylsulfonylbenzoesäuremethylester

1,9 mmol Olefin 3c) und 2,6 g AD-mix-α (J. Org. Chem. 1992, 57, S. 2769) werden in 9 ml t-Butanol und 9 ml H₂O 5 h bei RT gerührt. Dann werden 3 g Na₂SO₃ zugegeben, und es wird weitere 30 min bei RT gerührt. Anschließend wird 3 x mit je 50 ml EE extrahiet, über Na₂SO₄ getrocknet und das Solvens im Vakuum entfernt. Chromatographie an Kieselgel mit MTB liefert 130 mg farbloses Öl.
R_{f} (MTB) = 0,14 MS (ES, + LiCl) : 387 (M + 7)

### e) 4-[4-(1(R),2(R)-Dihydroxyprop-1-yl)phenoxy]-3-methylsulfonylbenzoylguanidin

0,29 mmol Methylester 3d und 1,5 mmol Guanidin werden nach der allgemeinen Vorschrift B umgesetzt. 29 mg, amorpher Feststoff.
R_{f} (EE/MeOH 5:1) = 0,23 MS (ES) : 408 (M + 1)

Die Titelverbindung des Beispiels 4 wird analog Beispiels 3 unter Verwendung von AD-mix-β synthetisiert.

### Beispiel 4

4-[4(1(S),2(S)-Dihydroxyprop-1-yl)phenoxy]-3-methylsulfonylbenzoylguanidin 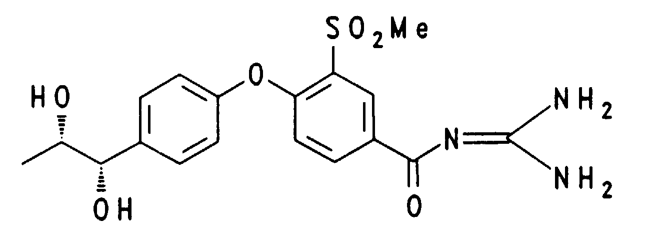 R_{f} (EE/MeOH 5:1) = 0,23 MS (ES) : 408 (M + 1)

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

| Inhibition des Na⁺/H⁺-Exchangers: | |
|---|---|
| Beispiel | IC₅₀ µmol/l |
| 1 | 0,4 |
| 2 | 0,4 |
| 3 | 0,3 - 0,5 |
| 4 | 0,1 - 0,5. |

## Patentansprüche

1. Benzoylguanidin der Formel I 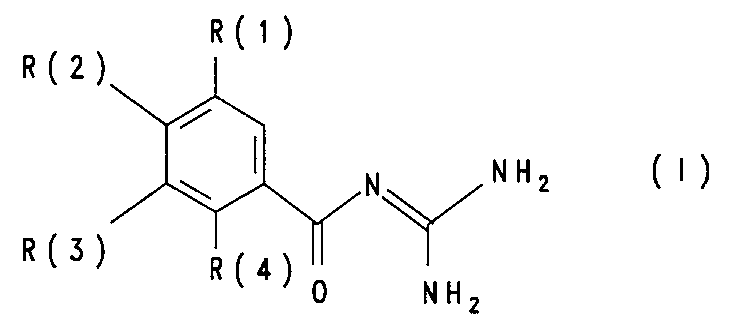 worin bedeuten:
R(1) Wasserstoff, F, Cl, Br, I, -NO₂, -C≡N, Xₒ-(CH₂)ₚ-(CF₂)_{q}-CF₃, R(5)-SOₘ, R(6)-CO- oder R(6)R(7)N-SO₂-, mit
X gleich Sauerstoff, S, NR(14),
m gleich Null, 1 oder 2,
o gleich Null, 1,
p gleich Null, 1, 2,
q gleich Null, 1, 2, 3, 4, 5, 6,
R(5) und R(6) gleich (C₁-C₈)-Alkyl, (C₃-C₆)-Alkenyl, -CₙH₂ₙ-R(8), CF₃,
n gleich Null, 1, 2, 3 oder 4,
R(8) gleich (C₃-C₇)-Cycloalkyl, Phenyl, welches nicht substituiert ist oder substituiert mit 1 - 3 Substituenten aus der Gruppe
F, Cl, CF₃, Methyl, Methoxy oder NR(9)R(10) mit R(9) und R(10) gleich H oder C₁-C₄-Alkyl,
wobei R(6) auch in der Bedeutung von H steht,
R(7) gleich H oder (C₁-C₄)-Alkyl,
wobei R(6) und R(7) gemeinsam 4 oder 5 Methylengruppen sein können, von denen eine CH₂-Gruppe durch Sauerstoff, S, NH,
N-CH₃ oder N-Benzyl ersetzt sein kann,
R(2) 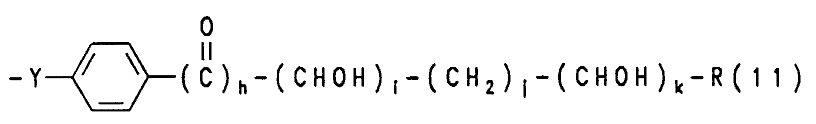 oder 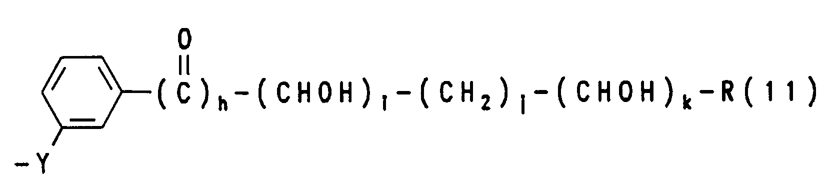 oder 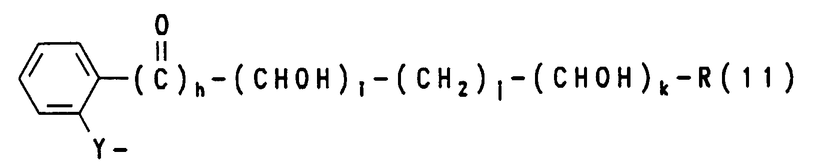
wobei Y gleich Sauerstoff, -S- oder -NR(12)- ist,
R(11), R(12) = Wasserstoff oder (C₁-C₃)-Alkyl, und
h Null oder 1, und
i, j und k unabhängig Null, 1, 2, 3 oder 4 bedeuten,
wobei jedoch nicht h, i und k gleichzeitig Null sind,
R(3) wie R(1) definiert, oder (C₁-C₆)-Alkyl, -X-R(13) mit
X gleich Sauerstoff, S, NR(14),
R(14) gleich H, (C₁-C₃)-Alkyl,
R(13) gleich H, (C₁-C₆)-Alkyl, (C₃-C₈)-Cycloalkyl, -C_{b}H_{2b}-R(15) mit b gleich Null, 1, 2, 3 oder 4 ist und
wobei R(13) und R(14) auch gemeinsam 4 oder 5 Methylengruppen sein können und eine CH₂-Gruppe durch Sauerstoff, S, NH, N-CH₃ oder N-Benzyl ersetzt sein kann,
R(15) Phenyl, das unsubstituiert oder substituiert ist mit 1-3
Substituenten aus der Gruppe F, Cl, CF₃, Methyl, Methoxy oder NR(9)R(10) mit
R(9) und R(10) gleich H oder (C₁-C₄)-Alkyl,
R(4) Wasserstoff, -OR(16) oder -NR(16)R(17) mit
R(16), R(17) unabhängig Wasserstoff, (C₁-C₃)-Alkyl
sowie deren pharmazeutisch verträgliche Salze.

2. Verbindung der Formel I nach Anspruch 1, in der bedeuten:
R(1) Wasserstoff, F, Cl, -C≡N, -CF₃, R(5)-SOₘ, R(6)-CO- oder
R(6)R(7)N-SO₂-, mit
m gleich Null, 1 oder 2,
R(5) und R(6) gleich (C₁-C₈)-Alkyl, (C₃-C₄)-Alkenyl, -CₙH₂ₙ-R(8), -CF₃,
n gleich Null oder 1,
R(8) gleich (C₃-C₆)-Cycloalkyl, Phenyl,
welches unsubstituiert ist oder substituiert mit 1-3 Substituenten aus der Gruppe F, Cl, CF₃, Methyl, Methoxy oder NR(9)R(10) mit R(9) und R(10) gleich H oder Methyl,
wobei R(6) auch in der Bedeutung von H steht,
R(7) gleich H oder Methyl.
R(3) Wasserstoff, Methyl, Cyano, -CF₃, F, Cl
und die übrigen Reste wie in Anspruch 1 definiert sind,
sowie deren pharmazeutisch verträgliche Salze.

3. Verbindung der Formel 1 nach Anspruch 1, in der bedeuten:
R(1) Wasserstoff, F, Cl, -C≡N, -CF₃, R(5)-SOₘ, R(6)-CO- oder
R(6)R(7)N-SO₂-, mit
m gleich Null, 1 oder 2,
R(5) gleich Methyl oder CF₃,
R(6), R(7) unabhängig voneinander gleich H oder Methyl;
R(2) 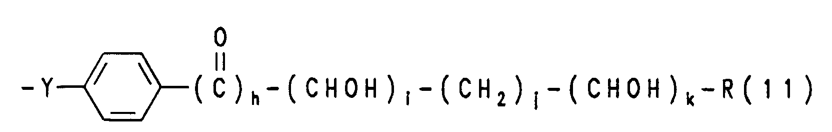 oder 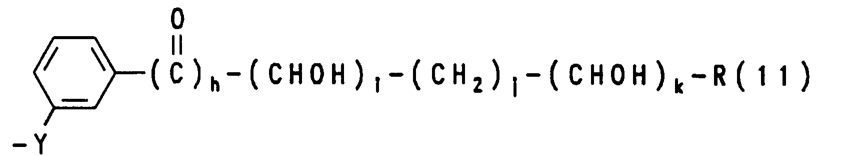 oder 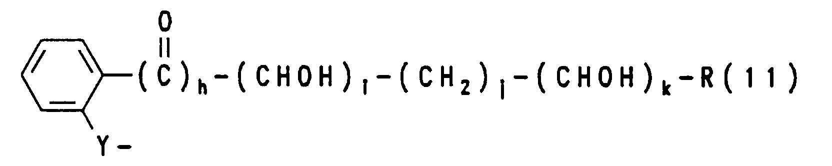
wobei Y = Sauerstoff, S oder -NR(12),
R(11), R(12) unabhängig Wasserstoff oder Methyl,
h gleich Null oder 1,
i, k unabhängig voneinander gleich Null, 1, 2 oder 3,
j gleich Null oder 1,
wobei jedoch nicht h, i und k gleichzeitig Null sind,
R(3) Methyl, Cyano, Trifluormethyl, F, Cl oder Wasserstoff,
R(4) Wasserstoff, OH oder NH₂
sowie deren pharmazeutisch verträgliche Salze.

4. Verfahren zur Herstellung einer Verbindung I nach Anspruch 1, dadurch gekennzeichnet, daß man
eine Verbindung der Formel II 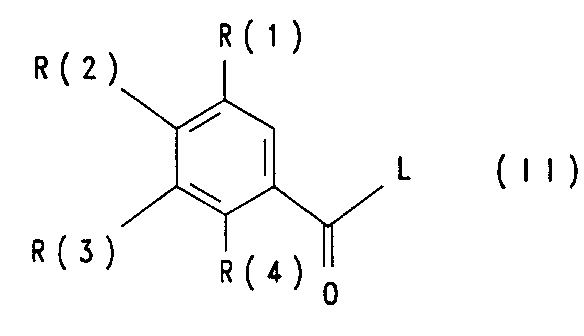 mit Guanidin umsetzt, worin R(1) bis R(4) die angegebene Bedeutung besitzen und L für eine nucleophil substituierbare Fluchtgruppe steht.

5. Verwendung einer Verbindung I nach Anspruch 1 zum Herstellen eines Medikaments zum Behandeln von Arrhythmien.

6. Verwendung einer Verbindung I nach Anspruch 1 zum Herstellen eines Medikaments zur Behandlung oder Prophylaxe des Herzinfarkts.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch I zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung 1 nach Anspruch 1 zur Herstellung eines Medikaments zur Behandlung von Schockzuständen.

12. Verwendung einer Verbindung I nach Anspruch I zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch 1 zur Herstellung eines Medikaments zur Behandlung vonKrankheiten, bei denendie Zellproliferation eien primäre oder sekundäre Ursache darstellt, und somit ihre Verwendung als Antiatherosklerotika, Mittel gegen diabetische Spätkomplikationen, Krebserkrankungen, fibrotische Erkrankungen wie Luingenfibrose, Leberfibrose oder Nierenfibrose, Prostatahyperplasie.

15. Verwendung einer Verbindung I nach Anspruch 1 zur Herstellung eines wissenschaftlichen Tools zur Inhibition des Na⁺/H⁺-Exchangers, zur Diagnose der Hypertonie und proliferativer Erkrankungen.

16. Heilmittel, enthaltend eine wirksame Menge einer Verbindung I nach Anspruch 1.

## Claims

1. A benzoylguanidine of the formula I 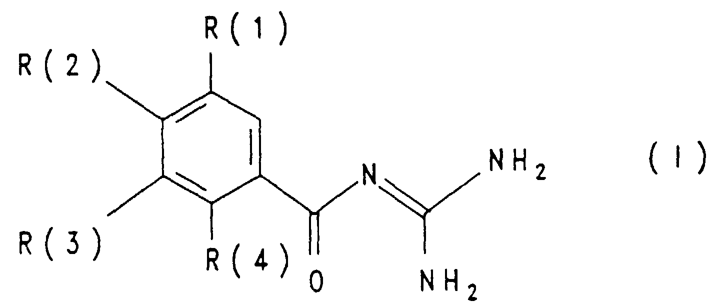 in which:
R(1) is hydrogen, F, Cl, Br, I, -NO₂, -C≡N, Xₒ-(CH₂)ₚ-(CF₂)_{q}-CF₃, R(5)-SOₘ, R(6)-CO- or R(6)R(7)N-SO₂-, where
X is oxygen, S or NR(14),
m is zero, 1 or 2,
o is zero or 1,
p is zero, 1 or 2,
q is zero, 1, 2, 3, 4, 5 or 6,
R(5) and R(6) are (C₁-C₈)-alkyl, (C₃-C₆)-alkenyl, -CₙH₂ₙ-R(8) or CF₃,
n is zero, 1, 2, 3 or 4,
R(8) is (C₃-C₇)-cycloalkyl or phenyl which is unsubstituted or substituted by 1-3 substituents from the group comprising
F, Cl, CF₃, methyl, methoxy and NR(9)R(10) where R(9) and R(10) are H or C₁-C₄-alkyl,
where R(6) also has the meaning of H,
R(7) is H or (C₁-C₄)-alkyl,
where R(6) and R(7) together can be 4 or 5 methylene groups, of which one CH₂ group can be replaced by oxygen, S, NH, N-CH₃ or N-benzyl,
R(2) is 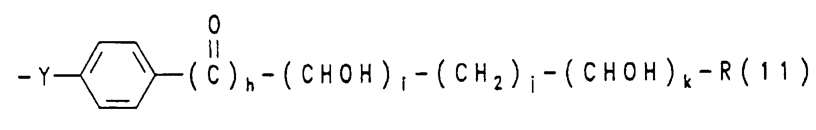 or 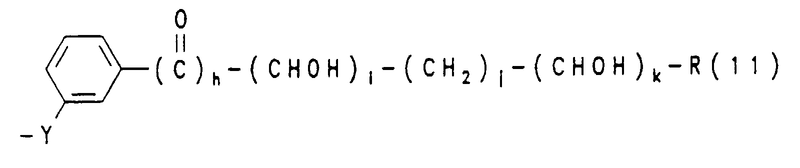 or 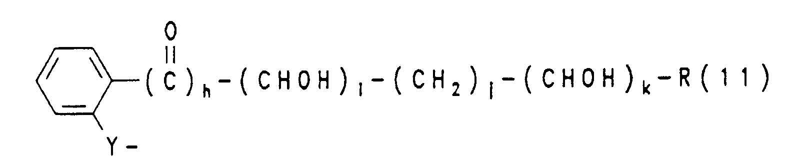
where Y is oxygen, -S- or -NR(12)-,
R(11) and R(12) = hydrogen or (C₁-C₃)-alkyl, and
h is zero or 1, and
i, j and k independently are zero, 1, 2, 3 or 4,
but where h, i and k are not simultaneously zero,
R(3) is defined as R(1), or is (C₁-C₆)-alkyl or -X-R(13) where
X is oxygen, S or NR(14),
R(14) is H or (C₁-C₃)-alkyl,
R(13) is H, (C₁-C₆)-alkyl, (C₃-C₈)-cycloalkyl or -C_{b}H_{2b}-R(15) where
b is zero, 1, 2, 3 or 4 and
where R(13) and R(14) together can also be 4 or 5 methylene groups and a CH₂ group can be replaced by oxygen, S, NH, N-CH₃ or N-benzyl,
R(15) is phenyl which is unsubstituted or substituted by 1-3 substituents from the group comprising F, Cl, CF₃, methyl, methoxy and NR(9)R(10) where R(9) and R(10) are H or (C₁-C₄)-alkyl,
R(4) is hydrogen, -OR(16) or -NR(16)R(17) where
R(16) and R(17) are independently hydrogen or (C₁-C₃)-alkyl
or its pharmaceutically tolerable salts.

2. A compound of the formula I as claimed in claim 1, in which:
R(1) is hydrogen, F, Cl, -C≡N, -CF₃, R(5)-SOₘ, R(6)-CO- or R(6)R(7)N-SO₂-, where
m is zero, 1 or 2,
R(5) and R(6) are (C₁-C₈)-alkyl, (C₃-C₄)-alkenyl; -CₙH₂ₙ-R(8) or -CF₃,
n is zero or 1,
R(8) is (C₃-C₆)-cycloalkyl or phenyl,
which is unsubstituted or substituted by 1-3 substituents from the group comprising F, Cl, CF₃, methyl, methoxy and NR(9)R(10) where R(9) and R(10) are H or methyl,
where R(6) also has the meaning of H,
R(7) is H or methyl,
R(3) is hydrogen, methyl, cyano, -CF₃, F or Cl
and the other radicals are as defined in claim 1,
or its pharmaceutically tolerable salts.

3. A compound of the formula I as claimed in claim 1, in which:
R(1) is hydrogen, F, Cl, -C≡N, -CF₃, R(5)-SOₘ,
R(6)-CO- or R(6)R(7)N-SO₂-, where
m is zero, 1 or 2,
R(5) is methyl or CF₃,
R(6) and R(7) independently of one another are H or methyl;
R(2) is 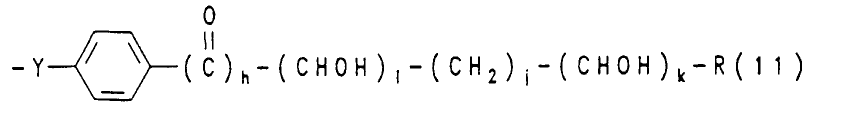 or 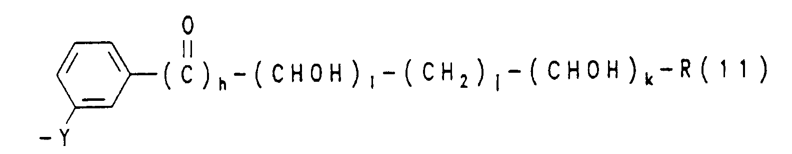 or 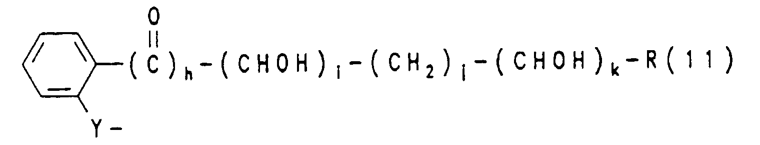
where Y = oxygen, S or -NR(12),
R(11) and R(12) independently are hydrogen or methyl,
h is zero or 1,
i and k independently of one another are zero, 1,
2 or 3,
j is zero or 1,
but where h, i and k are not simultaneously zero, R(3) is methyl, cyano, trifluoromethyl, F, Cl or hydrogen,
R(4) is hydrogen, OH or NH₂
or its pharmaceutically tolerable salts.

4. A process for the preparation of a compound I as claimed in claim 1, which comprises reacting a compound of the formula II 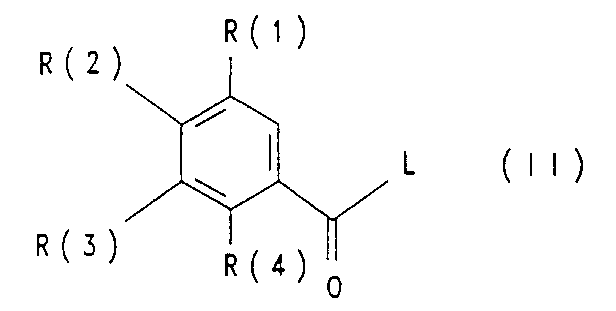 in which R(1) to R(4) have the given meaning and L is a leaving group which can be nucleophilically substituted, with guanidine.

5. The use of a compound I as claimed in claim 1 for the production of a medicament for the treatment of arrhythmias.

6. The use of a compound I as claimed in claim 1 for the production of a medicament for the treatment or prophylaxis of cardiac infarct.

7. The use of a compound I as claimed in claim 1 for the production of a medicament for the treatment or prophylaxis of angina pectoris.

8. The use of a compound I as claimed in claim 1 for the production of a medicament for the treatment or prophylaxis of ischemic conditions of the heart.

9. The use of a compound I as claimed in claim 1 for the production of a medicament for the treatment or prophylaxis of ischemic conditions of the peripheral and central nervous system and of stroke.

10. The use of a compound I as claimed in claim 1 for the production of a medicament for the treatment or prophylaxis of ischemic conditions of peripheral organs and members.

11. The use of a compound I as claimed in claim 1 for the production of a medicament for the treatment of states of shock.

12. The use of a compound I as claimed in claim 1 for the production of a medicament for use in surgical operations and organ transplants.

13. The use of a compound I as claimed in claim 1 for the production of a medicament for the preservation and storage of transplants for surgical measures.

14. The use of a compound I as claimed in claim 1 for the production of a medicament for the treatment of diseases in which cell proliferation is a primary or secondary cause, and thus their use as antiatherosclerotics, agents against diabetic late complications, cancers, fibrotic disorders such as pulmonary fibrosis, fibrosis of the liver or fibrosis of the kidneys, and prostate hyperplasia.

15. The use of a compound I as claimed in claim 1 for the production of a scientific tool for inhibition of the Na⁺/H⁺ exchanger, and for the diagnosis of hypertension and proliferative disorders.

16. A medicament containing an effective amount of a compound I as claimed in claim 1.

## Revendications

1. Benzoylguanidines de formule I 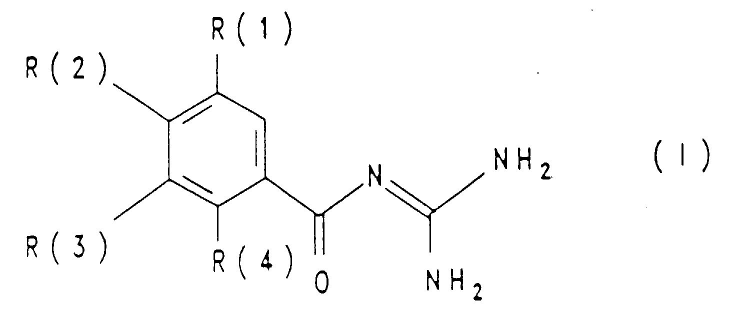 dans laquelle :
R(1) représente un atome d'hydrogène, F, CI, Br, I, un radical -NO₂, -C≡N, X₀-(CH₂)ₚ-(CF₂)_{q}-CF₃, R(5)-SOₘ, R(6)-CO- ou R(6)R(7)N-SO₂-, avec
X étant un atome d'oxygène, S, NR(14),
m zéro, ou 2,
o zéro, 1,
p zéro, 1 ou 2,
q zéro, 1, 2, 3, 4, 5, 6,
R(5) et R(6) un groupe alkyle en C₁-C₈,
alcényle en C₃-C₆, un groupe -CₙH₂ₙ-R(8), CF₃,
n étant zéro, 1, 2, 3 ou 4,
R(8) un groupe cycloalkyle en C₃-C₇, phényle, qui est non substitué ou substitué avec 1 - 3 substituants choisis parmi
F, Cl, CF₃, les groupes méthyle, méthoxy ou NR(9)R(10) avec
R(9) et R(10) étant un atome d'hydrogène ou un radical alkyle en C₁-C₄,
R(6) représentant également un atome d'hydrogène,
R(7) un atome d'hydrogène ou un radical alkyle en C₁-C₄,
R(6) et R(7) pouvant être ensemble 4 ou 5 groupes méthylène, parmi lesquels un groupe CH₂ peut être remplacé par un atome d'oxygène, S, un groupe NH, N-CH₃ ou N-benzyle,
R(2) 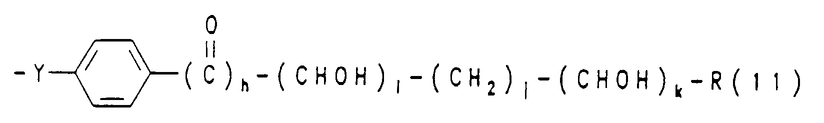 ou 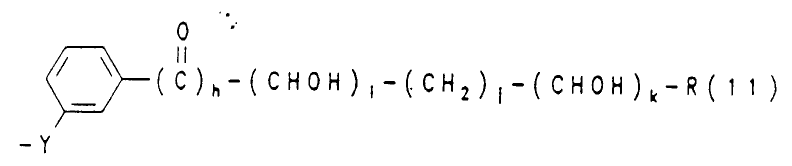 ou 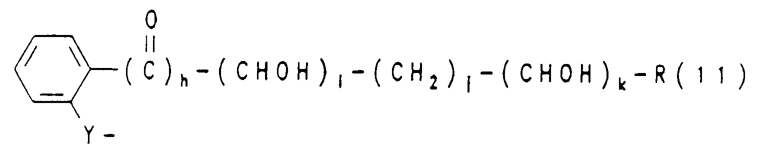
Y étant un atome d'oxygène, -S- ou un groupe -NR(12),
R(11), R(12) = un atome d'hydrogène ou un groupe alkyle en C₁-C₃, et
h zéro ou 1, et
i, j et k représentant indépendamment zéro, 1, 2, 3 ou 4, h, i et k n'étant pas nuls en même temps,
R(3) est défini comme R(1), ou est un groupe alkyle en C₁-C₆, -X-R(13) avec
X étant un atome d'oxygène, S, un groupe NR(14),
R(14) étant un atome d'hydrogène, un groupe alkyle en C₁-C₃,
R(13) étant un atome d'hydrogène, un groupe alkyle en C₁-C₆, cycloalkyle en C₃-C₈, -C_{b}H_{2b}-
R(15) avec b étant zéro, 1, 2, 3 ou 4 et
R(13) et R(14) pouvant être ensemble 4 ou 5 groupes méthylène et un groupe CH₂ pouvant être remplacé par un atome d'oxygène, S, un groupe NH, N-CH₃ ou N-benzyle,
R(15) un groupe phényle qui est non substitué ou substitué avec 1 - 3 substituants choisis parmi F, CI, CF₃, les groupes méthyle, méthoxy ou NR(9)R(10) avec
R(9) et R(10) étant un atome d'hydrogène ou un radical alkyle en C₁-C₄;
R(4) représente un atome d'hydrogène, un radical -OR(16) ou -NR(16)R(17) avec
R(16), R(17) étant indépendamment un atome d'hydrogène, un radical alkyle en C₁-C₃,
ainsi que leurs sels pharmacologiquement acceptables.

2. Composé de formule I selon la revendication 1, dans laquelle :
R(1) représente un atome d'hydrogène, F, CI, un radical -C≡N, R(5)-SOₘ, R(6)-CO- ou R(6)R(7)N-SO₂-, avec
m étant zéro, 1 ou 2,
R(5) et R(6) un groupe alkyle en C₁-C₈, alcényle en C₃-C₄, un groupe -CₙH₂ₙ-R(8), CF₃,
n étant zéro ou 1,
R(8) un groupe cycloalkyle en C₃-C₆, phényle,
qui est non substitué ou substitué avec 1 - 3 substituants choisis parmi F, Cl, CF₃, les groupes méthyle, méthoxy ou NR(9)R(10) avec R(9) et R(10) étant un atome d'hydrogène ou un radical méthyle,
R(6) représentant aussi un atome d'hydrogène,
R(7) étant un atome d'hydrogène ou un radical méthyle.
R(3) représente un atome d'hydrogène, un groupe méthyle, cyano, -CF₃, F, Cl
et les autres groupes sont comme définis ci-dessus,
ainsi que leurs sels pharmacologiquement acceptables.

3. Composé de formule I selon la revendication 1, dans laquelle :
R(1) représente un atome d'hydrogène, F, CI, un radical -C≡N, -CF₃, R(5)-SOₘ, R(6)-CO- ou R(6)R(7)N-SO₂-, avec
m étant zéro, 1 ou 2,
R(5) un groupe méthyle ou CF₃,
R(6), R(7) indépendamment l'un de l'autre un atome d'hydrogène ou un groupe méthyle;
R(2) 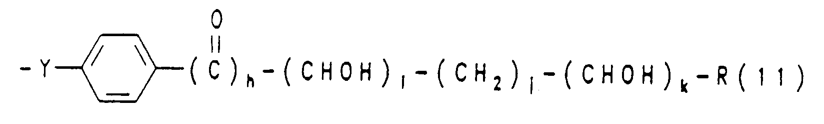 ou 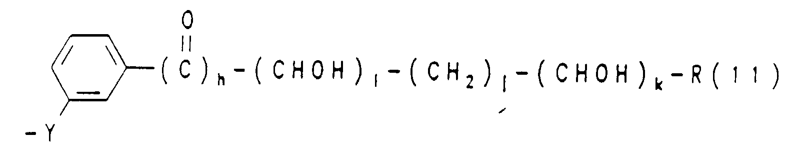 ou 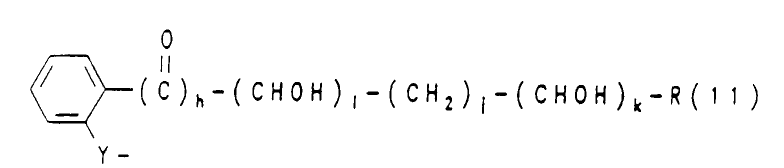
Y étant un atome d'oxygène, -S- ou un groupe -NR(12),
R(11), R(12) indépendamment un atome d'hydrogène ou un groupe méthyle,
h zéro ou 1,
i, et k indépendamment l'un de l'autre zéro, 1, 2 ou 3,
j zéro ou 1,
h, i et k n'étant cependant pas nuls en même temps,
R(3) un groupe méthyle, cyano, trifluorométhyle, un atome de fluor, de chlore ou d'hydrogène,
R(4) un atome d'hydrogène, un groupe OH ou NH₂
ainsi que leurs sels pharmaceutiquement acceptables.

4. Procédé de préparation d'un composé I selon la revendication 1, caractérisé en ce que l'on
fait réagir un composé de formule II 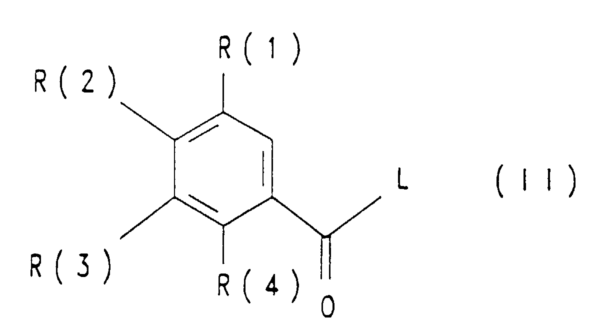 avec la guanidine, R(1) à R(4) ayant la signification indiquée et L représentant un groupe partant substituable par un nucléophile.

5. Utilisation d'un composé I selon la revendication 1 pour la préparation d'un médicament pour le traitement des arythmies.

6. Utilisation d'un composé I selon la revendication 1 pour la préparation d'un médicament pour le traitement ou la prophylaxie de l'infarctus.

7. Utilisation d'un composé I selon la revendication 1 pour la préparation d'un médicament pour le traitement ou la prophylaxie de l'angine de poitrine.

8. Utilisation d'un composé I selon la revendication 1 pour la préparation d'un médicament pour le traitement ou la prophylaxie des affections cardiaques dues aux ischémies

9. Utilisation d'un composé I selon la revendication 1 pour la préparation d'un médicament pour le traitement ou la prophylaxie des atteintes du système nerveux périphérique et central dues aux ischémies et de l'apoplexie.

10. Utilisation d'un composé I selon la revendication 1 pour la préparation d'un médicament pour le traitement ou la prophylaxie des affections ischémiques des organes périphériques et des membres.

11. Utilisation d'un composé I selon la revendication 1 pour la préparation d'un médicament pour le traitement d'états de choc.

12. Utilisation d'un composé I selon la revendication 1 pour la préparation d'un médicament à utiliser au cours des opérations chirurgicales et des transplantations d'organes.

13. Utilisation d'un composé I selon la revendication 1 pour la préparation d'un médicament pour la conservation et le stockage des transplants pour des opérations chirurgicales.

14. Utilisation d'un composé I selon la revendication 1 pour la préparation d'un médicament pour le traitement de maladies dans lesquelles la prolifération cellulaire représente une cause primaire ou secondaire, et par conséquent son utilisation comme antiathérosclérotique, comme agent contre les complications tardives du diabète, les maladies cancéreuses, les maladies fibrotiques comme la fibrose du poumon, la fibrose du foie ou la fibrose des reins, l'hyperplasie de la prostate.

15. Utilisation d'un composé I selon la revendication 1 pour la préparation d'un outil efficace d'inhibition de l'échangeurs Na⁺/H⁺, pour le diagnostic de l'hypertonie et des maladies prolifératives.

16. Médicament contenant une quantité efficace d'un composé I selon la revendication 1.
